# EUROPÄISCHE PATENTSCHRIFT

(11) **EP 3 845 209 B1**
(45) Veröffentlichungstag und Bekanntmachung des Hinweises auf die Patenterteilung: **07.06.2023**
(21) Anmeldenummer: 21158592.2
(22) Anmeldetag: 03.04.2019
(51) Int. Cl.: A61F 9/007, A61B 17/22

(54) **PHACO-EMULSIFIKATIONSHANDSTÜCK**
PHACOEMULSIFICATION HANDPIECE
PIÈCE À MAIN POUR PHACO-ÉMULSIFICATION

(30) Priorität: 04.04.2018 WO PCT/EP2018/058600
(43) Veröffentlichungstag der Anmeldung: 07.07.2021
(62) Teilanmeldung aus: 19720373.0
(73) Patentinhaber: Wefis GmbH, 50996 Köln (DE)
(72) Erfinder: WERNZ, Dietrich, 50996 Köln (DE); WÖRSDÖRFER, Stefan, 50996 Köln (DE)
(74) Vertreter: V.O.

(56) Entgegenhaltungen:
- EP-A1- 2 011 458
- WO-A1-2007/070081
- US-A1- 2014 271 251
- US-A1- 2014 276 364
- US-A1- 2017 281 216
- US-B2- 7 431 728

## Beschreibung

Die Erfindung betrifft ein Phaco-Emulsifikationshandstück, bei dem es sich vornehmlich um ein Einmal-Instrument handelt.

Phaco-Emulsifikationshandstücke werden als chirurgische Instrumente in der Augenchirurgie eingesetzt. Beispiele für derartige chirurgische Instrumente sind in WO-A-93/15703-A, WO-A-00/53136-A, EP-A-2 011 458, DE-A-102 09 495 und DE-C-4 008 594 beschrieben.

In WO-A-89/06515 ist eine Vorrichtung für die In-Vivo-Angioplastie beschrieben, die eine Sonotrode aus einer Aluminiumlegierung aufweist, bei der es sich um AI-2024 oder AI-7075 handeln kann.

Medizinische Ultraschallvorrichtungen mit Wellenleitern aus verschiedenen Materialien, wie z.B. Titan, Eisen oder Stahl, sind aus WO-A-2007/070081 bekannt.

Weitere medizinische Ultraschallvorrichtungen sind aus US-B-7 431 728 bekannt.

Ein Phaco-Emulsifikationshandstück dient zum Zertrümmern der Augenlinse und Absaugen von Geweberesten. Zu diesem Zweck ist das Werkzeug als Hohlnadel ausgebildet und wird von einem Vibrationsmechanismus des Handstücks, der als Ultraschallwandler ausgeführt ist, in Hochfrequente lineare Vor- und Zurückbewegungen versetzt. Außen um die Hohlnadel herum wird eine Spülflüssigkeit, beispielsweise eine Emulsionsflüssigkeit zugeführt, die zur Operationsstelle gelangt und von dort zusammen mit herausgelösten Gewebebestandteilen durch das Werkzeug und einen Absaugkanal des Handstücks abgesaugt wird.

Die Kosten für die Herstellung eines Phaco-Emulsifikationshandstücks sind nicht unbeträchtlich und werden im Wesentlichen bestimmt durch den Ultraschallwandler und das Gehäuse. Aufgrund der vergleichsweise hohen Herstellungskosten werden daher Phaco-Emulsifikationshandstücke mehrfach verwendet.

Daher müssen die Einzelkomponenten aus vergleichsweise langlebigen Materialien bestehen. Für den Mehrfacheinsatz eines Phaco-Emulsifikationshandstücks ist dessen Sterilisation nach jedem Gebrauch bzw. vor einem weiteren Gebrauch zwingend erforderlich. Auch dies ist mit zusätzlichen Kosten verbunden und überdies problematisch, weshalb bereits darüber nachgedacht wurde, Phaco-Emulsifikationshandstücke für den Einmalgebrauch zu entwickeln. Dies scheiterte bisher allerdings an den weiterhin recht hohen Herstellungskosten.

Aus US-A-2014/271251 ist ein Phaco-Emulsifikationshandstück nach dem Oberbegriff des Anspruchs 1 bekannt.

Ein weiteres bekanntes Phaco-Emulsifikationshandstück ist in US-A-2014/0276364 beschrieben. Ein weiteres chirurgisches Instrument ist in US-A-2017/0281216 beschrieben.

Aufgabe der Erfindung ist es daher, die Einzelkomponenten eines Phaco-Emulsifikationshandstücks, weiter zu vereinfachen, um kostengünstiger herstellen zu können.

Zur Lösung dieser Aufgabe wird mit der Erfindung ein Phaco-Emulsifikationshandstück vorgeschlagen, das versehen ist mit den Merkmalen des Anspruchs 1. Einzelne Ausgestaltungen der Erfindung sind Gegenstand der Unteransprüche.

Nach der Erfindung weist das Phaco-Emulsifikationshandstück ein Gehäuse aus oder mit Kunststoff auf. Ferner ist erfindungsgemäß vorgesehen, dass das Gehäuse ein zumindest einen Teil der Sonotrode umgebendes Innengehäuseteil und ein Außengehäuseteil aufweist, zwischen denen sich ein Ringraum für die Zuführung und/oder für die Weiterleitung von Spülflüssigkeit zum Werkzeug befindet. Wie bereits oben erwähnt, könnte die Kühlung der Sonotrode durch die durch den Ringraum strömende Spülflüssigkeit bei einem Kunststoff-Innengehäuseteil problematisch sein. Dies hängt beispielsweise von der Dicke des Innengehäuseteils ab, wobei anzumerken ist, dass aus Stabilitätsgründen ein gewisses Dickenmaß für das Innengehäuseteil nicht unterschritten werden sollte. Damit ergibt sich möglicherweise die Schwierigkeit, dass das Innengehäuseteil zu dickwandig ist, um aufgrund seiner Umspülung durch die Spülflüssigkeit die Sonotrode noch ausreichend kühlen zu können. Daher weist das Innengehäuseteil des erfindungsgemäßen Phaco-Emulsifikationshandstücks Kühlöffnungen auf, innerhalb derer die Sonotrode und insbesondere der Mittelabschnitt der Sonotrode freiliegt und zur Kühlung mit Spülflüssigkeit in Kontakt gelangt.

Würde man nun die Sonotrode aus einem Aluminiumwerkstoff herstellen, wie es mit Vorteil bei dem erfindungsgemäßen Phaco-Emulsifikationshandstück der Fall sein kann, so stellt sich hier das Problem der möglicherweise nicht mehr gegebenen Biokompatibilität. Daher müsste man durch beispielweise eine entsprechende Beschichtung dafür Sorge tragen, dass der Aluminiumwerkstoff zumindest in denjenigen Bereichen der Sonotrode, in denen diese innerhalb der Kühlöffnungen freiliegt, nicht mit Spülflüssigkeit in Kontakt gelangt.

In weiterer vorteilhafter Ausgestaltung der Erfindung kann vorgesehen sein, dass das Innengehäuseteil eine Austrittsöffnung für das vordere Ende der Sonotrode aufweist und dass das Außengehäuseteil eine mit der Austrittsöffnung des Innengehäuseteils fluchtende und von dieser in Längserstreckung der Sonotrode beabstandete Austrittsöffnung aufweist, in der das vordere Ende der Sonotrode angeordnet ist und/oder über die das vordere Ende der Sonotrode übersteht und auf die ein Halteelement aufsetzbar, insbesondere aufsteck- oder aufschraubbar ist, von dem aus sich eine elastische Hülse erstreckt, die das Werkzeug von außen umgibt. Das Außengehäuseteil steht am vorderen Ende des Phaco-Emulsifikationshandstücks also über das Innengehäuseteil über. Durch die Austrittsöffnung des Innengehäuseteils ragt die Sonotrode aus dem Innengehäuseteil heraus, und zwar insbesondere mit ihrem verschlankten vorderen Ende. Dieses vordere Ende erstreckt sich dann weiter durch das Außengehäuseteil und durch dessen insbesondere als Stutzen ausgebildete Austrittsöffnung bzw. es endet in dieser Austrittsöffnung. Damit schließt sich also an den Ringraum zwischen den beiden Gehäuseteilen ein weiterer Ringraum an, der durch das Außengehäuseteil und die Sonotrode bzw. deren vorderes Ende definiert ist. Auch in diesem Bereich kommt es nun zu einem Kontakt der Spülflüssigkeit mit der Sonotrode. Das Material der Sonotrode sollte also biokompatibel sein. Wenn als Material eine Aluminiumlegierung eingesetzt wird, wie es gemäß der Erfindung vorgeschlagen wird, und wenn es sich herausstellen sollte, dass dieses Material nicht biokompatibel ist, so müsste man also beispielsweise die Sonotrode in diesem Bereich mit biokompatiblem Material beschichten. Alternativ kann selbstverständlich auch der sich durch den dem Innengehäuseteil vorgelagerten Ringraum erstreckende Teil der Sonotrode aus einem anderen metallischen Material als Aluminium bzw. einer Aluminiumlegierung bestehen. Dann hätte man sozusagen eine Sonotrode, die aus verschiedenen metallischen Materialien besteht. Der besagte Teil der Sonotrode, der nicht aus Aluminium bzw. einer Aluminiumlegierung besteht, kann beispielsweise aus Titan, Eisen oder Stahl oder einem anderen biokompatiblen Metall bestehen, das den beim Betrieb des Ultraschallwandlers auftretenden Biegewellen standhält.

In weiterer zweckmäßiger Ausgestaltung der Erfindung kann vorgesehen sein, dass sich an den Ringraum zwischen dem Innengehäuseteil und dem Außengehäuseteil an der Austrittsöffnung des Innengehäuseteils ein weiterer Ringraum anschließt, der von dem Außengehäuseteil und dem vorderen Ende der Sonotrode gebildet ist und der sich bis in den durch das Spiel zwischen dem vorderen Ende der Sonotrode und der Austrittsöffnung des Außengehäuseteils definierten Zwischenraum erstreckt und von dort in einen Zwischenraum zwischen dem Werkzeug und der elastischen Hülse übergeht.

Wie bereits oben beschrieben, ist der Ultraschallwandler in dem Gehäuse des Phaco-Emulsifikationshandstücks angeordnet und dort gelagert. Zweckmäßig ist es diesbezüglich, wenn die Sonotrode zwei axial beabstandete Aufnahmenuten für jeweils einen Lagerring, insbesondere für einen elastischen Lagerring und vorzugsweise für einen O-Ring aufweist, wobei die beiden Lagerringe zur Lagerung des Ultraschallwandlers von innen an dem Gehäuse und insbesondere an dem Innengehäuseteil anliegen.

Alternativ zur zuvor genannten Lagerung und Zentrierung der Sonotrode in dem Gehäuse bzw. in dem Innengehäuseteil kann vorgesehen sein, dass die Sonotrode an ihrem dem vorderen Ende abgewandten hinteren Ende eine Aufnahmenut für einen Lagerring, insbesondere für einen elastischen Lagerring und vorzugsweise für einen O-Ring, aufweist und dass das vordere Ende der Sonotrode durch nach innen vorstehende Rippen o.dgl. Vorsprünge eines Stutzens des Gehäuses zentriert ist.

Es ist von Vorteil, wenn die Kühlöffnungen in dem zwischen den beiden Lagerringen liegenden Bereich des Gehäuses des Innengehäuseteils angeordnet sind, wobei die beiden Aufnahmenuten in dem Mittelabschnitt der Sonotrode ausgebildet sind. Die Lagerungsringe dichten also den Bereich beidseitig der Anordnung der Kühlöffnungen ab. Sie dienen damit nicht nur der Lagerung, sondern verhindern auch das Eindringen von Spülflüssigkeit in das Innengehäuseteil.

Zweckmäßig ist es ferner, dass das Gehäuse eine in den Ringraum mündende Einlassöffnung für die Zufuhr von Spülflüssigkeit aufweist.

Vorn Vorteil kann es sein, wenn das Gehäuse an seinem dem vorderen Ende der Sonotrode abgewandten hinteren Ende eine Durchführöffnung für einen Absaugschlauch aufweist, der mit dem sich durch die Sonotrode hindurch erstreckenden Absaugkanal verbunden ist.

In weiterer vorteilhafter Ausgestaltung der Erfindung kann aus dem Gehäuse ein Anschlusskabel herausgeführt werden, dass an das Piezokeramikelement angeschlossen ist.

Die Erfindung wird nachfolgend anhand zweier Ausführungsbeispiele sowie unter Bezugnahme auf die Zeichnung näher erläutert. Im Einzelnen zeigen dabei:
- Fig. 1: einen Längsschnitt durch ein Phaco-Emulsifikationshandstück,
- Fig. 2: einen Querschnitt entlang der Linie II-II der Fig. 1,
- Fig. 3: eine erste Seitenansicht auf das Phaco-Emulsifikationshandstück gemäß Fig. 1 mit teilweise aufgebrochenem und im Schnitt dargestelltem Gehäuse,
- Fig. 4: eine weitere Seitenansicht des Phaco-Emulsifikationshandstücks mit ebenfalls teilweise aufgebrochenem, jedoch in Seitenansicht gezeigtem Innengehäuseteil,
- Fig. 5: eine Seitenansicht des Ultraschallwandlers des Phaco-Emulsifikationshandstück,
- Fig. 6: einen Längsschnitt durch den Ultraschallwandler gemäß Fig. 5,
- Fig. 7: einen Längsschnitt durch ein alternativ ausgebildetes Phaco-Emulsifikationshandstück und
- Fig. 8: einen Querschnitt entlang der Linie VIII-VIII der Fig. 7.

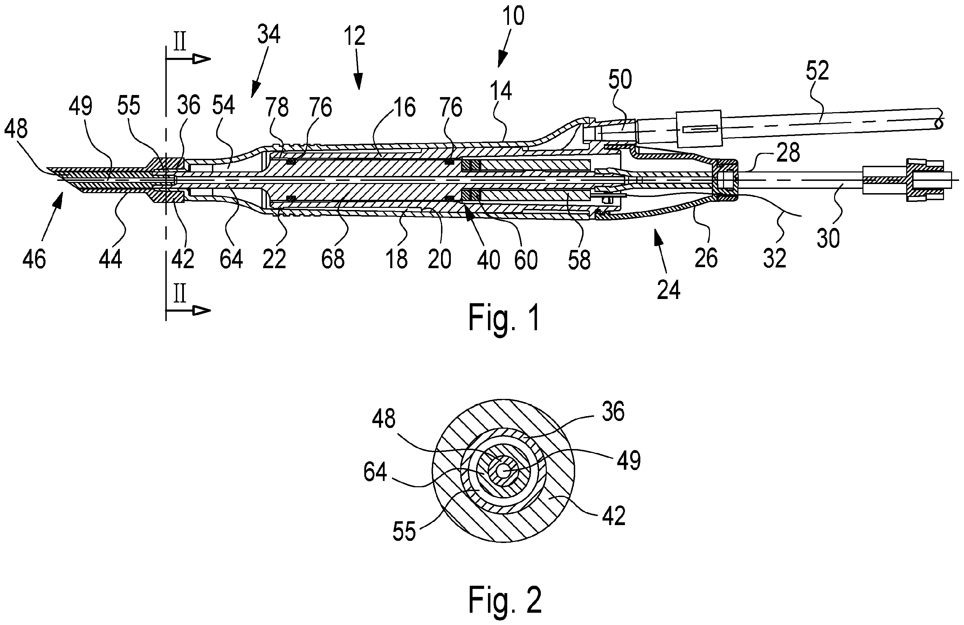

Die Fign. 1 bis 4 zeigen verschiedene Darstellungen eines Phaco-Emulsifikationshandstück 10 gemäß einem ersten Ausführungsbeispiel der Erfindung. Das Handstück 10 weist ein Gehäuse 12 aus Kunststoff auf. Dieses Gehäuse umfasst einen Außengehäuseteil 14 und einen im Außengehäuseteil 14 angeordneten Innengehäuseteil 16. Zwischen beiden Gehäuseteilen 14,16 bildet sich ein erster Ringraum 18, zu dem das Innengehäuseteil 16 durch mehrere Außenvorsprünge 20, 22 zentrisch gelagert ist. Am hinteren Ende 24 des Gehäuses 12 ist dieses durch ein- oder mehrteiliges Deckelteil 26 abgeschlossen, aus dem über eine Durchführöffnung 28 ein Absaugschlauch 30 herausgeführt ist. Ferner ist aus dem Deckelteil 26 ein Anschlusskabel 32 herausgeführt. Auf diese beiden Komponenten wird später noch eingegangen werden.

Wie anhand der Fign. 1 bis 4 zu erkennen ist, überragt das Außengehäuseteil 14 an dem vorderen Ende 34 des Gehäuses 12 das Innengehäuseteil 16. Am vorderen Ende 34 des Gehäuses 12 befindet sich ein Stutzen 36, der eine Austrittsöffnung 38 für einen im Gehäuse 12 angeordneten Ultraschallwandler 40 bildet. Auf den Stutzen 36 ist ein Halteelement 42 aufgeschraubt, an dem sich eine elastische Hülse 44 befindet, die ein Werkzeug 46 in Form einer Hohlnadel 48 (mit Lumen 49) des Handstücks 10 umgibt.

Über eine Einlassöffnung 50, an die ein Spülflüssigkeitsschlauch 52 angeschlossen ist, gelangt Sprühflüssigkeit in den Ringraum 18 des Gehäuses 12, die aus dem Ringraum 18 heraus in einen weiteren Ringraum 54 gelangt und von diesem aus der Austrittsöffnung 38 in den Zwischenraum zwischen Werkzeug 46 und elastischer Hülse 44 gelangt.

Im Innengehäuseteil 16 des Gehäuses 12 befindet sich der bereits oben angesprochene Ultraschallwandler 40 des Phaco-Emulsifikationshandstücks 10.

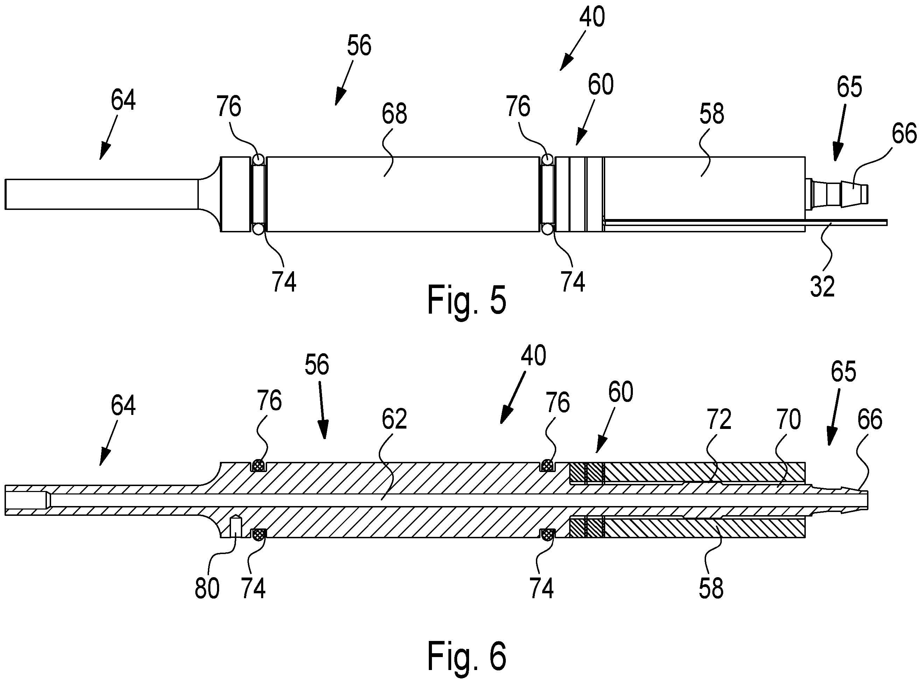

Wie anhand der Fign. 5 und 6 zu erkennen ist, umfasst der Ultraschallwandler 40 eine Sonotrode 56, einen Resonator 58 und mindestens ein Piezokeramikelement 60. An das Piezokeramikelement 60 ist das Anschlusskabel 32 angeschlossen. Durch die Sonotrode 56 hindurch erstreckt sich ein Absaugkanal 62, der an dem vorderen Ende 64 der Sonotrode 56 in Fluidverbindung mit dem Lumen der Hohlnadel 48 steht und an dessen hinterem Ende 65 ein Anschlussstutzen 66 zum Anschluss des Absaugschlauchs 30 vorgesehen ist. Die Sonotrode 56 weist einen Mittelabschnitt 68 auf, gegenüber dem das vordere Ende 64 verschlankt ist. Die Spülflüssigkeit gelangt durch einen Zwischenraum 55 zwischen dem Stutzen 36 und dem vorderen Ende 64 der Sonotrode 56 (siehe Fig. 2), mit dem das Werkzeug 46 verbunden ist, und weiter zwischen das Werkzeug 46 und die elastische Hülse 44, die sich dementsprechend aufweitet, wenn das Werkzeug 46 von Spülflüssigkeit umgeben ist.

Dem vorderen Ende 64 gegenüberliegend weist die Sonotrode 56 ein ebenfalls verschlanktes hinteres Endstück 70 auf, das sich durch den als Hohlzylinder ausgebildeten Resonator 58 erstreckt und mit diesem bei 72 in Gewindeeingriff steht. Auf diese Weise kann das mindestens eine Piezokeramikelement 60 zwischen Resonator 58 und Mittelabschnitt 68 der Sonotrode 56 eingespannt gehalten werden. Der Mittelabschnitt 68 der Sonotrode ist mit zwei Umfangsaufnahmenuten 74 für jeweils einen elastischen Lagerring 76 versehen, über die die Sonotrode 56 und damit der Ultraschallwandler 40 im Innengehäuseteil 16 des Gehäuses 12 gelagert ist.

Die Besonderheit der hier beschriebenen Sonotrode 56 ist in der Auswahl ihres Material zu sehen. Erfindungsgemäß wird eine hochfeste Aluminiumlegierung eingesetzt, deren Zugfestigkeit größer als 400 N/mm² und insbesondere größer als 450 N/mm² ist.

Wie anhand von Fig. 1 zu erkennen ist, umgibt das Innengehäuseteil 16 den Mittelabschnitt 68 der Sonotrode 56 sowie das Piezokeramikelement 60 und den Resonator 58. Im Bereich des Übergangs vom Mittelabschnitt 68 zum verschlankten vorderen Ende 64 ragt die Sonotrode 56 aus dem Innengehäuseteil 16 heraus. An die insoweit am Innengehäuseteil 16 vorgesehene Austrittsöffnung 78 schließt sich der weitere Ringraum 54 an, der definiert ist durch das Außengehäuseteil 14 und das verschlankte vordere Ende 64 der Sonotrode 56.

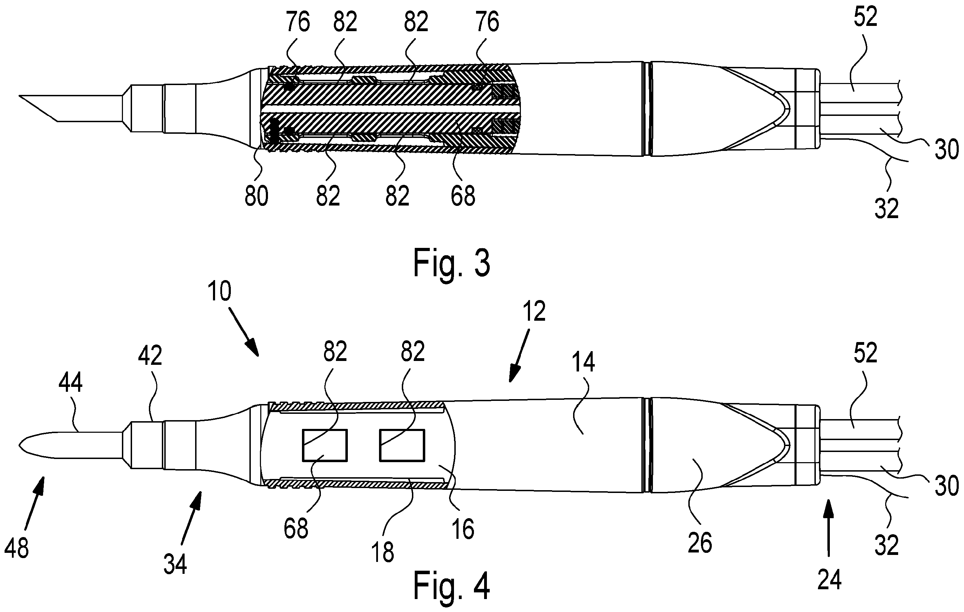

Wie weiter oben bereits erwähnt, handelt es sich bei dem Gehäuse 12 um ein Gehäuse aus Kunststoff. Die Sonotrode 56 ist in dem Kunststoffinnengehäuseteil 16 gelagert, und zwar über die beiden Lagerringe 76. Bei 80 ist in den Fign. 3 und 6 angedeutet, dass der Ultraschallwandler 40 im Bereich seiner Sonotrode 56 mit dem Kunststoffinnengehäuseteil 16 verstiftelt ist. Beim Betrieb des Ultraschallwandlers 40 erhitzt sich die Sonotrode 56 merklich, weshalb sie gekühlt werden sollte. Dies erfolgt durch die Spülflüssigkeit, wozu das Kunststoff-Innengehäuseteil 16 im Bereich um den Mittelabschnitt 68 der Sonotrode 56 herum mit mehreren Kühlöffnungen 82 versehen ist (siehe Fign. 3 und 4). Innerhalb dieser Kühlöffnungen 82 liegt die Sonotrode 56 mit ihrem Mittelabschnitt 68 frei, so dass nun die Spülflüssigkeit in diesen Bereichen an der Sonotrode 56 direkt entlangströmen kann, wodurch der Kühleffekt wesentlich effektiver ist als wenn das Kunststoffinnengehäuseteil 16 durchgehend ausgeführt wäre. Dadurch, dass nun die Spülflüssigkeit, die während der Operation mit dem zu behandelnden Gewebe in Kontakt kommt, an der Sonotrode 56 in deren Mittelabschnitt 68 und in deren verschlanktem Ende 64 (nämlich im Ringraum 54) entlangströmt, sollte dafür gesorgt werden, dass das Material der Sonotrode biokompatibel ist. Bei einer Aluminiumlegierung und einer Spülflüssigkeit, die als Kochsalzlösung ausgeführt ist, ist dies möglicherweise nicht in ausreichendem Maße gegeben. Daher sollte die Sonotrode 56 zumindest in den zuvor genannten Bereichen mit einer biokompatiblen Beschichtung versehen sein. Alternativ kann aber auch das gesamte vordere Ende 64 der Sonotrode 56 aus einem biokompatiblen Metall wie beispielsweise Titan bestehen. Dieser Teil der Sonotrode 56 würde dann mit dem restlichen Teil der Sonotrode 56 durch beispielsweise Verschraubung mechanisch verbunden sein.

Wie anhand von Fig. 3 zu erkennen ist, befinden sich die Lagerringe 76 beidseitig der Anordnung der Kühlöffnungen 82 und dichten somit die Sonotrode 56 insbesondere gegenüber dem Resonator 58 und dem Piezokeramikelement 60 ab, so dass in diesen Bereich keine Spülflüssigkeit gelangen kann.

Die Merkmale des Kunststoffgehäuses und einzelne Varianten davon sind in den nachfolgenden Merkmalsgruppen aufgeführt, die in Kombination miteinander treten können, bzw. auch einzeln selbsttätig und in all diesen Fällen die Erfindung und/oder Ausgestaltungen der Erfindung definieren.

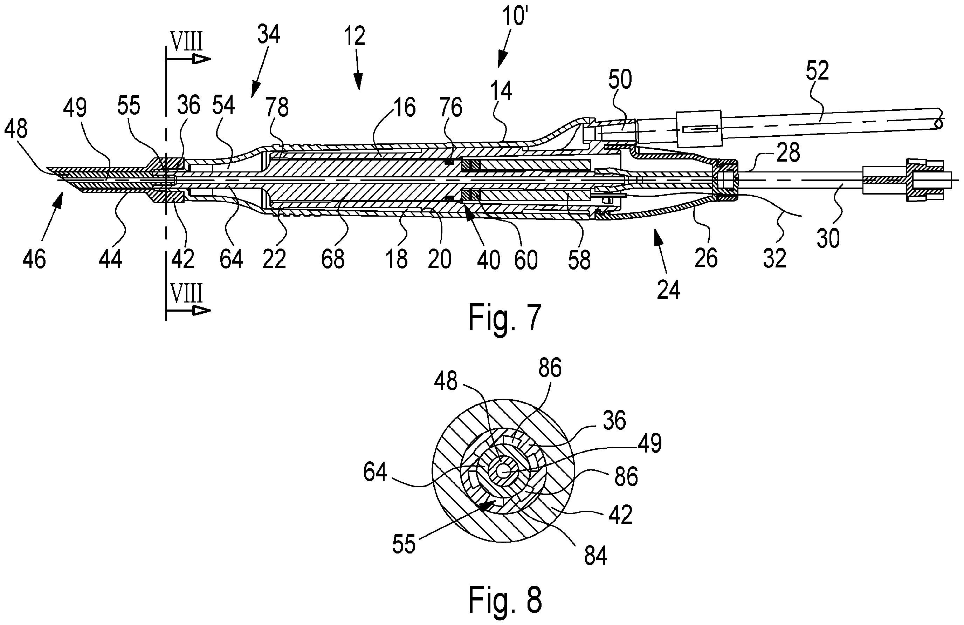

Anhand der Fign. 7 und 8 wird nachfolgend auf eine alternative Ausgestaltung der zentrischen Lagerung der Sonotrode im Gehäuse des Handstücks eingegangen, das in diesen Figuren mit 10' bezeichnet ist. Soweit die einzelnen Elemente des Handstücks 10' der Fign. 7 und 8 konstruktiv oder funktional denjenigen des Handstücks 10 der Fign. 1 bis 6 entsprechen, sind sie in den Fign. 7 und 8 mit den gleichen Bezugszeichen wie in den Fign. 1 bis 6 gekennzeichnet.

Der Unterschied beider Handstücke 10 und 10' ist in der Lagerung der Sonotrode 56 zu sehen. Im Handstück 10' ist die Sonotrode 56 an ihrem hinteren Ende 65 bzw. in ihrem Mittelabschnitt durch den O-Ring 76 gelagert, während ihr vorderes Ende 64 an nach innen vorstehenden Rippen 84 des Stutzens 36 des Außengehäuseteils 14 zentriert ist (siehe Fig. 8). Die Rippen 84 ragen in den Zwischenraum 55 hinein und unterteilen diesen in Einzelkanäle 86. Die Rippen 84 versteifen zudem den Stutzen 36 und damit das Gehäuse. Die vordere Aufnahmenut 74 der Sonotrode 56, die bei dem Handstück 10 der Fign. 1 bis 6 vorhanden ist, kann entfallen, was fertigungs- und montagetechnisch (der vordere O-Ring und dessen Montage an der Sonotrode entfallen) vorteilhaft ist.

### Bezugszeichenliste

- 10: (Phaco-Emulsifikations-)Handstück
- 10': (Phaco-Emulsifikations-)Handstück
- 12: Gehäuse
- 14: Außengehäuseteil
- 16: Innengehäuseteil
- 18: Ringraum
- 20: Außenvorsprünge
- 22: Außenvorsprünge
- 24: (hinteres) Ende des Gehäuses
- 26: Deckelteil
- 28: Durchführöffnung
- 30: Absaugschlauch
- 32: Anschlusskabel
- 34: (vorderes) Ende des Gehäuses
- 36: Stutzen
- 38: Austrittsöffnung für die Sonotrode am Außengehäuseteil
- 40: Ultraschallwandler
- 42: Halteelement
- 44: elastische Hülse (sleeve)
- 46: Werkzeug
- 48: Hohlnadel
- 49: Lumen der Hohlnadel
- 50: Einlassöffnung
- 52: Spülflüssigkeitsschlauch
- 54: Ringraum
- 55: Zwischenraum zwischen dem Stutzen und dem vorderen Ende der Sonotrode
- 56: Sonotrode
- 58: Resonator
- 60: Piezokeramikelement
- 62: Absaugkanal
- 64: vorderes Ende der Sonotrode
- 65: hinteres Ende der Sonotrode
- 66: Anschlussstutzen
- 68: Mittelabschnitt
- 70: Endstück
- 72: Gewindeeingriff zwischen Sonotrode und Resonator
- 74: Umfangsaufnahmenuten
- 76: Lagerring
- 78: Austrittsöffnung für die Sonotrode am Innengehäuseteil
- 80: Verstiftelung
- 82: Kühlöffnungen
- 84: (Führungs- und Versteifungs-)Rippen
- 86: Kanäle

## Patentansprüche

1. Phaco-Emulsifikationshandstück mit
- einem Gehäuse (12),
- einem Ultraschallwandler (40), der in dem Gehäuse (12) angeordnet ist, und
- einem insbesondere Metall wie z.B. Stahl, Eisen oder Titan aufweisenden Werkzeug (46), das zwecks Ultraschallanregung mit dem Ultraschallwandler (40) in Wirkverbindung steht,
- wobei der Ultraschallwandler (40) eine Sonotrode (56), einen Resonator (58) und ein zwischen diesen angeordnetes Piezokeramikelement (60) aufweist,
- wobei das Gehäuse (12) Kunststoff aufweist und/oder aus Kunststoff besteht, **dadurchgekennzeichnet,**
- dass das Gehäuse (12) ein zumindest einen Teil der Sonotrode (56) umgebendes Innengehäuseteil (16) und ein Außengehäuseteil (14) aufweist, zwischen denen sich ein Ringraum (18) für die Zuführung und/oder für die Weiterleitung von Spülflüssigkeit zum Werkzeug (46) befindet, und
- dass das Innengehäuseteil (16) Kühlöffnungen (82) aufweist, innerhalb der die Sonotrode (56) und insbesondere der Mittelabschnitt (68) der Sonotrode (56) freiliegt und zur Kühlung mit Spülflüssigkeit in Kontakt gelangt.

2. Phaco-Emulsifikationshandstück nach Anspruch 1, **dadurch gekennzeichnet, dass** das Innengehäuseteil (16) eine Austrittsöffnung (78) für das vordere Ende (34) der Sonotrode (56) aufweist und dass das Außengehäuseteil (14) eine mit der Austrittsöffnung (38) des Innengehäuseteils (16) fluchtende und von dieser in Längserstreckung der Sonotrode (56) beabstandete Austrittsöffnung (78) aufweist, in der das vordere Ende (34) der Sonotrode (56) angeordnet ist und/oder über die das vordere Ende (34) der Sonotrode (56) übersteht und auf die ein Halteelement (42) aufsetzbar, insbesondere aufsteck- oder aufschraubbar ist, von dem aus sich eine elastische Hülse (44) erstreckt, die das Werkzeug (46) von außen umgibt.

3. Phaco-Emulsifikationshandstück nach Anspruch 2, **dadurch gekennzeichnet, dass** sich an den Ringraum (18) zwischen dem Innengehäuseteil (16) und dem Außengehäuseteil (14) an der Austrittsöffnung (78) des Innengehäuseteils (16) ein weiterer Ringraum (54) anschließt, der von dem Außengehäuseteil (14) und dem vorderen Ende (64) der Sonotrode (56) gebildet ist und der sich bis in den durch ein Spiel zwischen dem vorderen Ende (34) der Sonotrode (56) und der Austrittsöffnung (38) des Außengehäuseteils (14) definierten Zwischenraum (55) erstreckt und von dort in einen Zwischenraum zwischen dem Werkzeug (46) und der elastischen Hülse (44) übergeht.

4. Phaco-Emulsifikationshandstück nach einem der Ansprüche 1 bis 3, **dadurch gekennzeichnet, dass** die Sonotrode (56) zwei axial beabstandete Aufnahmenuten (74) für jeweils einen Lagerring (76), insbesondere für einen elastischen Lagerring und vorzugsweise für einen O-Ring aufweist, wobei die beiden Lagerringe zur Lagerung des Ultraschallwandlers (40) von innen an dem Gehäuse (12) und insbesondere an dem Innengehäuseteil (16) anliegen.

5. Phaco-Emulsifikationshandstück nach einem der Ansprüche 1 bis 3, **dadurch gekennzeichnet, dass** die Sonotrode (56) an ihrem dem vorderen Ende (64) abgewandten hinteren Ende (65) oder in ihrem mittleren Abschnitt eine Aufnahmenut (74) für einen Lagerring (76), insbesondere für einen elastischen Lagerring und vorzugsweise für einen O-Ring, aufweist und dass das vordere Ende (64) der Sonotrode (56) durch nach innen vorstehende Rippen (84) o.dgl. Vorsprünge eines Stutzens (36) des Gehäuses (12) zentriert ist.

6. Phaco-Emulsifikationshandstück nach einem der Ansprüche 1 bis 3 oder nach einem der Ansprüche 4 bis 7, sofern auf Anspruch 3 rückbezogen, **dadurch gekennzeichnet, dass** die Kühlöffnungen (82) in dem zwischen den beiden Lagerringen liegenden Bereich des Gehäuses (12) bzw. des Innengehäuseteils (16) angeordnet sind und dass die beiden Aufnahmenuten (74) in dem Mittelabschnitt (68) der Sonotrode (56) ausgebildet sind.

7. Phaco-Emulsifikationshandstück nach einem der Ansprüche 1 bis 6, **dadurch gekennzeichnet, dass** das Gehäuse (12) eine mit dem Ringraum (18) in Fluidverbindung stehende Einlassöffnung (50) für die Zufuhr von Spülflüssigkeit aufweist.

8. Phaco-Emulsifikationshandstück nach einem der Ansprüche 1 bis 7, **dadurch gekennzeichnet, dass** das Gehäuse (12) an seinem dem vorderen Ende (34) der Sonotrode (56) abgewandten hinteren Ende (24) eine Durchführöffnung (28) für einen Absaugschlauch (30) aufweist, der mit dem sich durch die Sonotrode (56) hindurch erstreckenden Absaugkanal (62) verbunden ist.

9. Phaco-Emulsifikationshandstück nach einem der Ansprüche 1 bis 8, **dadurch gekennzeichnet, dass** aus dem Gehäuse (12) ein Anschlusskabel (32) herausgeführt ist, das an das Piezokeramikelement (60) angeschlossen ist.

10. Phaco-Emulsifikationshandstück nach einem der Ansprüche 1 bis 9, **dadurch gekennzeichnet, dass** das Werkzeug (46) eine Hohlnadel (48) ist.

## Claims

1. A phacoemulsification handpiece comprising
- a housing (12),
- an ultrasonic transducer (40), which is arranged in the housing (12), and
- a tool (46) comprising in particular metal such as e.g. steel, iron or titanium, which is in operative connection with the ultrasonic transducer (40) for the purpose of ultrasonic excitation,
- wherein the ultrasonic transducer (40) has a sonotrode (56), a resonator (58), and a piezoceramic element (60) arranged between them,
- wherein the housing (12) comprises plastic and/or consists of plastic, **characterised in**
- **that** the housing (12) comprises an inner housing part (16) surrounding at least one part of the sonotrode (56), and an outer housing part (14), between which there is an annular space (18) for the supply and/or for the transmission of flushing liquid to the tool (46), and
- **that** the inner housing part (16) has cooling openings (82), within which the sonotrode (56) and in particular the middle section (68) of the sonotrode (56) is exposed and comes into contact with flushing liquid for cooling.

2. The phacoemulsification handpiece according to claim 1, **characterised in that** the inner housing part (16) has an outlet opening (78) for the front end (34) of the sonotrode (56), and that the outer housing part (14) has an outlet opening (78) aligned with the outlet opening (38) of the inner housing part (16) and spaced apart from the latter in the longitudinal extension of the sonotrode (56), in which the front end (34) of the sonotrode (56) is arranged and/or over which the front end (34) of the sonotrode (56) protrudes and onto which a retaining element (42) can be placed, in particular can be attached or screwed on, from which an elastic sleeve (44) extends, which surrounds the tool (46) from the outside.

3. The phacoemulsification handpiece according to claim 2, **characterised in that** a further annular space (54) is connected to the annular space (18) between the inner housing part (16) and the outer housing part (14) at the outlet opening (78) of the inner housing part (16), which is formed by the outer housing part (14) and the front end (64) of the sonotrode (56) and which extends into the intermediate space (55) defined by the clearance between the front end (34) of the sonotrode (56) and the outlet opening (38) of the outer housing part (14) and from there passes into an intermediate space between the tool (46) and the elastic sleeve (44).

4. The phacoemulsification handpiece according to any one of claims 1 to 3, **characterised in that** the sonotrode (56) has two axially spaced receiving grooves (74) for in each case one bearing ring (76), in particular for an elastic bearing ring and preferably for an O-ring, wherein the two bearing rings for mounting the ultrasonic transducer (40) abut from the inside against the housing (12) and in particular against the inner housing part (16).

5. The phacoemulsification handpiece according to any one of claims 1 to 3, **characterised in that** the sonotrode (56) has a receiving groove (74) on its rear end (65) facing away from the front end (64) or in its middle section for a bearing ring (76), in particular for an elastic bearing ring and preferably for an O-ring and that the front end (64) of the sonotrode (56) is centred by inwardly projecting ribs (84) or similar projections of a connecting piece (36) of the housing (12).

6. The phacoemulsification handpiece according to any one of claims 1 to 3, or according to any one of claims 4 to 7, insofar as it relates back to claim 3, **characterised in that** the cooling openings (82) are arranged in the area of the housing (12) or of the inner housing part (16) between the two bearing rings and that the two receiving grooves (74) are formed in the middle section (68) of the sonotrode (56).

7. The phacoemulsification handpiece according to any one of claims 1 to 6, **characterised in that** the housing (12) has an inlet opening (50) in fluid communication with the annular space (18) for the supply of flushing liquid.

8. The phacoemulsification handpiece according to any one of claims 1 to 7, **characterised in that** the housing (12) has a feed-through opening (28) on its rear end (24) facing away from the front end (34) of the sonotrode (56) for a suction hose (30), which is connected to the suction channel (62) extending through the sonotrode (56).

9. The phacoemulsification handpiece according to any one of claims 1 to 8, **characterised in that** a connection cable (32) is led out of the housing (12), which connection cable is connected to the piezoceramic element (60).

10. The phacoemulsification handpiece according to any one of claims 1 to 9, **characterised in that** the tool (46) is a hollow needle (48).

## Revendications

1. Pièce à main de phaco-émulsification avec
- un logement (12),
- un transducteur à ultrasons (40) qui est agencé dans le logement (12), et
- un outil (46), notamment en métal tel que l'acier, le fer ou le titane, qui est fonctionnellement relié au transducteur à ultrasons (40) à des fins d'excitation à ultrasons,
- dans lequel le transducteur à ultrasons (40) comporte une sonotrode (56), un résonateur (58) et un élément piézocéramique (60) disposés entre eux,
- dans lequel le logement (12) présente du plastique et/ou est constitué de matière plastique,
**caractérisé en ce que**
- le logement (12) présente une partie de logement interne (16) entourant au moins une partie de la sonotrode (56) et une partie de logement externe (14), entre lesquelles se trouve un espace annulaire (18) pour l'alimentation et/ou pour l'acheminement du liquide de rinçage vers l'outil (46), et
- la partie intérieure du logement (16) présente des ouvertures de refroidissement (82), à l'intérieur desquelles la sonotrode (56) et en particulier la section médiane (68) de la sonotrode (56) est exposée et entre en contact avec le liquide de rinçage pour le refroidissement.

2. Pièce à main de phaco-émulsification selon la revendication 1, **caractérisée en ce que** la partie intérieure de logement (16) présente une ouverture de sortie (78) pour l'extrémité avant (34) de la sonotrode (56) et que la partie extérieure de logement (14) a une ouverture de sortie (38) de la partie intérieure de logement (16) et a une ouverture de sortie (78) qui est alignée et espacée de celle-ci dans le prolongement longitudinal de la sonotrode (56), dans lequel l'extrémité avant (34) de la sonotrode (56) est agencée et/ou à travers laquelle l'extrémité avant (34) de la sonotrode (56) fait saillie et sur laquelle un élément de maintien (42) peut être placé, notamment enfiché ou vissé, à partir duquel un manchon élastique (44) s'étend, qui entoure l'outil (46) de l'extérieur.

3. Pièce à main de phaco-émulsification selon la revendication 2, **caractérisée en ce qu'**un autre espace annulaire (54) jouxte l'espace annulaire (18) entre la partie intérieure du logement (16) et la partie extérieure du logement (14) au niveau de l'ouverture de sortie (78 ) de la partie intérieure de logement (16), qui est formé par la partie extérieure de logement (14) et l'extrémité avant (64) de la sonotrode (56) et qui s'étend jusqu'à un espace défini (55) par l'intervalle entre l'extrémité avant (34) de la sonotrode (56) et l'ouverture de sortie (38) de la partie de logement externe (14) et de là se transforme en un espace entre l'outil (46) et le manchon élastique (44).

4. Pièce à main de phaco-émulsification selon l'une des revendications 1 à 3, **caractérisée en ce que** la sonotrode (56) comporte deux gorges de réception (74) espacées axialement pour une bague d'appui respective (76), notamment pour une bague d'appui élastique et de préférence pour un joint torique, dans lequel les deux bagues d'appui pour le montage du transducteur à ultrasons (40) reposent sur le logement (12) et en particulier sur la partie intérieure du logement (16) depuis l'intérieur.

5. Pièce à main de phaco-émulsification selon l'une des revendications 1 à 3, **caractérisée en ce que** la sonotrode (56) présente une gorge de réception (74) pour une bague d'appui (76), notamment pour une bague d'appui élastique et de préférence pour un joint torique, et que l'extrémité avant (64) de la sonotrode (56) est centrée par des nervures en saillie vers l'intérieur (84) ou analogue d'une douille (36) du logement (12).

6. Pièce à main de phaco-émulsification selon l'une des revendications 1 à 3 ou selon l'une des revendications 4 à 7, si dépendante de la revendication 3, **caractérisée en ce que** les orifices de refroidissement (82) sont situés au niveau du logement (12) ou de la partie intérieure du logement (16) et que les deux rainures de réception (74) sont formées dans la section médiane (68) de la sonotrode (56).

7. Pièce à main de phaco-émulsification selon l'une des revendications 1 à 6, **caractérisée en ce que** le logement (12) présente une ouverture d'entrée (50) en communication fluidique avec l'espace annulaire (18) d'alimentation en liquide de rinçage.

8. Pièce à main de phaco-émulsification selon l'une des revendications 1 à 7, **caractérisée en ce que** l'extrémité arrière (24) du logement (12) opposée à l'extrémité avant (34) de la sonotrode (56) a une ouverture de passage (28) pour un tuyau d'aspiration (30) qui est relié au canal d'aspiration (62) s'étendant à travers la sonotrode (56).

9. Pièce à main de phaco-émulsification selon l'une quelconque des revendications 1 à 8, **caractérisée en ce qu'**un câble de liaison (32) est conduit hors du logement (12) et est relié à l'élément piézocéramique (60).

10. Pièce à main de phaco-émulsification selon l'une quelconque des revendications 1 à 9, **caractérisée en ce que** l'outil (46) est une aiguille creuse (48).
